# EUROPEAN PATENT APPLICATION

(11) **EP 4 349 356 A1**
(43) Date of publication of application: **10.04.2024**
(21) Application number: 23201467.0
(22) Date of filing: 03.10.2023
(51) Int. Cl.: A61K 36/185, A23L 33/00, A61K 8/00, A61K 9/00, A61K 31/00

(54) **METHOD FOR THE PREPARATION OF EXTRACTS ENRICHED WITH SATURATED, MONOUNSATURATED AND POLYUNSATURATED LONG-CHAIN FATTY ACIDS FROM TABEBUIA IMPETIGINOSA (LAPACHO)**

(30) Priority: 06.10.2022 IT 202200020613
(71) Applicant: Phytoitalia SRL, 20124 Milano (IT)
(72) Inventor: Volpi, Nicola, 41125 Modena (IT); Abbiati, Ezio, 25081 Bedizzole (BS) (IT)
(74) Representative: Rastelli, Franco

(57) **Abstract**

The invention refers to extracts, compositions and formulations rich in longchain fatty acids, in particular unsaturated ones, isolated from the bark and inner parts of the bark, from the leaves, the flowers, the buds and other parts of *Tabebuia impetiginosa* (*Lapacho* or *Pau d'arco*) and their structural characterization for food, nutraceutical, dietary use. More particularly, the invention refers to compositions comprising fractions isolated and purified from the bark and inner parts thereof of *Tabebuia impetiginosa* by a specific process of extraction and particularly rich in monounsaturated and polyunsaturated fatty acids defined omega and essential.

## Description

### BACKGROUND OF THE INVENTION

The present invention refers to extracts, compositions and formulations rich in long-chain fatty acids, in particular unsaturated, isolated from the bark and inner parts of the bark, from leaves, flowers, buds and other parts of *Tabebuia impetiginosa (Lapacho* or *Pau d'arco)* and to their structural characterization for food, nutraceutical, dietary use. More particularly, the invention refers to compositions comprising fractions isolated and purified from the bark and inner parts thereof of *Tabebuia impetiginosa* by a specific process of extraction and particularly rich in monounsaturated and polyunsaturated fatty acids defined omega and essential.

Historically, human beings used natural products such as plants, animals, microorganisms and other biological resources for alleviating and treating different diseases. Many of the drugs commercially available today have been originally developed from plants and other biological resources used in the traditional medicine. Therefore, the knowledge of the traditional use of natural products plays an important role in the discovery and development of drugs.

*Tabebuia impetiginosa* is a plant belonging to the family of Bignoniaceae, which is mainly present in the Amazonian rain forest and in other tropical regions of Central and Latin America and it is not only a decorative plant, but it has a high pharmaceutical value too. In fact, *T. impetiginosa* has been used as a traditional healing method for the treatment of various diseases and it has anti-nociceptive, anti-edematogenic, antibiotic and antidepressant properties. In addition, the inner bark of this tree may be transformed in concentrated powders or extracts (for example aqueous ones) for the treatment of various inflammatory skin diseases. Several categories of bioactive molecular compounds have been isolated and identified from *T. impetiginosa,* mainly quinones, flavonoids, naphthoquinones and benzoic acids. In the last few years, many studies and researches have shown that extracts or compounds isolated from *T*. *impetiginosa* reveal a wide range of pharmacological properties such as anti-obesity, antimycotic, anti-psoriatic, antioxidant, anti-inflammatory and antitumour activity. In addition, extracts of *Tabebuia* have proven to be particularly important in immunopharmacology.

*T. impetiginosa* is also known as *Handroanthus impetiginosus* as accepted on the website www.theplantlist.org. There are 17 valid synonyms for *Handroanthus impetiginosus. T. impetiginosa* is a member of the family Bignoniaceae, genus *Tabebuiae* species *impetiginosa* as written in NCBI: txid429701. Its genus name derives from a native language of Brazil, while the species name derives from the Latin word *"impetigo"* (impetigo), a common and highly contagious cutaneous infection. It was so called as people believed that this plant could be used for treating impetigo.

*T. impetiginosa* is also known as pink trumpet tree or purple trumpet tree for the colour of its flowers. In English it is called Ipe, Taheebo (ant wood) and purple tabebuia. In French it is known as Poui, while its Spanish names include Lapacho negro, lapacho and quebracho. In Deutsch, the common name is Lapachobaum, Trompetenbaum and Feenkraut. In Portuguese, this plant is recognized as Pau d'arco (bow tree), Ipe-roxo (red thick bark) and Ipe. *T*. *impetiginosa* is well known because of its conspicuous appearance. This deciduous species may reach a height of 30 meters and sheds its leaves during the dry season. The palmately compound and serrated leaves are green and arranged in opposite or subopposite pairs. The leaf shape is elliptic or oblong with pinnate venation. Showy purple, dark pink or pink flowers appear in spring. The calyx is campanulate, tubular with five lobes and is trumpet shaped. Its fruit is contained in a pod and is comprised of an elongated cylindrical capsule with thin seeds.

*T. impetiginosa* is a tree that can be found in South and Central America and in some parts of North America. Although it is best known for its presence in the Amazon rain forest, it is also found in Argentina, Bolivia, Brazil, Colombia, Costa Rica, Ecuador, El Salvador, French Guyana, Guatemala, Honduras, Mexico, Nicaragua, Panama, Paraguay, Peru, Suriname, Trinidad and Tobago, and Venezuela.

Preparations and extracts from *T. impetiginosa* have been traditionally used for treating cancer, obesity, depression, viral, fungal and bacterial infections and inflammatory symptoms such as pain, arthritis, colitis and prostatitis since the Inca civilization. The Callawaya Tribe makes a concentrated tea out of the inner bark of the tree for the treatment of inflammatory skin diseases. In addition, it can be used as an astringent and diuretic. The Caribbean folk healers utilize the bark and the leaves of *T. impetiginosa* to cure tooth ache, back pain and sexually transmitted diseases. Latino and Haitian populations were reported to use this plant for the treatment of infectious diseases. Brazilian people have traditionally used this plant for anti-inflammatory and analgesic purposes. Such ethnomedicinal uses of *T. impetiginosa* led scientists and experts in the field to pay attention to it to better understand its pharmacological and therapeutic properties for the possible future development of drugs and active substances.

Several categories of phytochemicals have been identified in the leaves, in the bark and wood of *T. impetiginosa.* From the bark 19 glycosides comprised of four iridoid glycosides, two lignan glycosides, two isocoumarin glycosides, three phenylethanoid glycosides, and eight phenolic glycosides were methanol-extracted. The major constituents of *T. impetiginosa* are furanonaphthoquinones, naphthoquinones, anthraquinones (e.g., anthraquinone-2-carboxylic acid), quinones, benzoic acid, flavonoids, cyclopentene dialdehydes, coumarins, iridoids and glycosidic derivatives of phenolic acids. The presence of naphthoquinones attracted scientific attention, with lapachol and beta-lapachone especially piquing the interest of professionals in the medical field. Lapachol inhibits the proliferation of cancer cells, while beta-lapachone exhibits strong toxicity to cells in animal models and in human beings.

Fatty acids too, in particular oleic acid, palmitic acid and linoleic acid, are found in the bark of *T. impetiginosa* as well as free sugars were identified in the bark, with glucose being the most abundant, followed by fructose and sucrose. Organic acids, especially oxalic acid, are present, as well as the fat-soluble alcohols alpha-tocopherol and gamma-tocopherol. In addition, *T. impetiginosa* has some volatile constituents that exhibit antioxidant activity, among which 4-methoxybenzaldehyde, 4-methoxyphenol, 5-allyl-1,2,3-trimethoxybenzene, 1-methoxy-4-(1E)-1-propenylbenzene and 4-methoxybenzyl alcohol. Cyclopentene derivatives are secondary metabolites of plants and *T. impetiginosa* contains six cyclopentenyl esters known as avallaneine A-F, two new cyclopentyl esters, avallaneine G and H, and two known cyclopentenyl esters. These cyclopentene derivatives may provide a significant anti-inflammatory effect by blocking the production of NO and PGE2. In addition, Koyama et al. isolated two cyclopentene dialdehydes, 2-formyl-5-(40-methoxy-benzoyl-oxy)-3-methyl-2-cyclopentene-1-acetaldehyde and the 2-formyl-5-(30,40-dimethoxybenzoyloxy)-3-methyl-2-cyclopentene-1-acetaldehyde, that exert anti-inflammatory activity in human leukocytes.

Fatty acids, both free and forming part of complex lipids, play numerous key roles in the metabolism: they constitute the main metabolic fuels (energy storage and transport), as essential components of all membranes and as gene regulators. In addition, dietary lipids provide polyunsaturated fatty acids (PUFAs) which are precursors of powerful metabolites with local action, that is the eicosanoids. As part of complex lipids, fatty acids are important also for thermal and electrical insulation, and for mechanical protection. In addition, the fatty acids and their derived salts may act as detergents and soaps owing to their amphipathic properties and for their ability to generate micelles.

Most of saturated fatty acids are linear hydrocarbon chains with an even number of carbon atoms. Most common fatty acids contain 12-22 carbon atoms. Vice versa, the monounsaturated fatty acids have double carbon-carbon bonds, that can occur in different positions. The most common ones have a chain length of 16-22 carbons and a double bond in cis configuration. The polyunsaturated fatty acids (PUFAs) show more than a double bond. The PUFAs, produced only by plants and by phytoplankton, are essential for all higher organisms, including mammals and fishes. The so-called omega-3 and omega-6 fatty acids, for example, cannot be interconverted, and both are essential nutrients.

Fatty acids represent 30-35% of total energy intake in many industrialized countries and the most important dietary sources of fatty acids are vegetable oils, dairy products, meat products, cereals and oily fishes or fish oils. The most common saturated fatty acid in animals, plants and microorganisms is palmitic acid (C16:0). Stearic acid (C18:0) is a fatty acid important in animals and in some mushrooms, and a minor component in almost all plants. Myristic acid (C14:0) has a widespread presence, occasionally as a major component. The shorter chain saturated fatty acids with 8-10 carbon atoms are found in the triacylglycerols of milk and coconut. Oleic acid (C18:1, omega-9) is the most common monosaturated fatty acid in plants and animals. It is found in microorganisms too. Palmitoleic acid (C16:1) is also widely found in animals, plants and microorganisms, and it is a major component in some seed oils. Linoleic acid (C18:2, omega-6) is one of the main fatty acids in plants. Arachidonic acid (C20:4, omega-6) is a major component of membrane phospholipids in the entire animal kingdom, but very little is found in the diet. Alpha-linolenic acid (C18:3, omega-3) is found in higher plants (soybean and rapeseed oil, seed oils) and sea weeds. Eicosapentaenoic acid (EPA; C20:5, omega-3) and docosahexaenoic acid (DHA; C22:6, omega-3) are the primary fatty acids of sea weeds, oily fishes and fish oils.

The substitution of saturated fats with monounsaturated and polyunsaturated fats reduces plasma concentration of total and LDL cholesterol. In addition to the effects on plasma lipids, the unsaturated fatty acids affect the metabolism and the immunological and cardiovascular events in many ways. For example, the long-chain and unsaturated fatty acids may have various beneficial effects on the cardiovascular system. In addition, they decrease platelet and leukocyte reactivity, inhibit the proliferation of lymphocytes and they slightly decrease the blood pressure. Further, the unsaturated fatty acids can also positively affect the characteristics and rheology of the blood vessel wall, prevent ventricular arrhythmias and improve insulin sensitivity. They also have many beneficial effects with regard to cardiovascular diseases. The unsaturated fatty acids are important for foetal growth and development, in particular for the central nervous system, affecting visual acuity and cognitive functions.

From many epidemiological and experimental studies, there is relatively strong evidence that there are significant beneficial effects of the additional intake of unsaturated fatty acids in general and PUFAs in particular. Many of the measurable effects on risk factors are observed following intakes of 1-2 g per day of very long chain unsaturated fatty acids with a significant benefit for better health for a longer time.

Fatty acids are also widely used as inactive ingredients (excipients) in pharmaceutical preparations and in the use of lipidic formulations. The largest amount of lipids used in pharmaceutical products is in the production of fatty emulsions, mainly for healthcare nutrition but also as carriers of drugs. Other important applications are in the formulation of liposomes, particles useful in lipid transport for other active ingredients. In addition, there has been an increase in the use of lipids as ingredients in several formulations owing to their biofunctional effects and their biocompatible nature. In addition, fatty acids themselves or as part of lipid complexes, are frequently used in cosmetics as soaps, emulsions and liposomes.

Several inventions are aimed at the properties of bioactive components of *Tabebuia.* For example, the publication US 2005/0245599 A1 describes a procedure to make beta-lapachone more soluble in pharmaceutically acceptable solvents useful for the formulation of new pharmaceutical compositions. The therapeutically effective amount of beta-lapachone, or a derivative or analogue thereof, is complexed with a pharmaceutically acceptable solubilising carrier molecule in aqueous solution.

The publication WO 2017/060535 A1 relates to a topical herbal formulation which is particularly suitable for the treatment of wounds and a method for its manufacturing. More specifically, it relates to a formulation based on herbs for the healing of topical wounds which includes Pau d'Arco too. The formulation based on herbs is particularly effective in the treatment of wounds and it is also recommended for the treatment of general skin disorders in humans, among which eczema and dermatitis.

The patent application ITBZ20100033A1, "Soluzione idroalcolica di fitocomplessi per uso odontostomatologico a base di *lapacho* e mastice di Chios e procedimento per la preparazione della stessa" (Hydroalcoholic solution of phytocomplexes for odontostomatologic use based on *lapacho* and mastic of Chios and method for the preparation thereof), concerns the use of non-toxic plant extracts in hydroalcoholic solution for the preparation of odontostomatological medicines. More particularly, it relates to the application of a series of phytocomplexes, consisting of the extracts of Lapacho and Mastic of Chios with specific properties in the field of oral hygiene. The synergistic activity of such products turns out to be particularly effective in antibacterial, antiviral and anti-inflammatory action of buccal cavity and also and above all in the protection of dental enamel from acid attack.

The patent US 5455033 A reports a medicinal composition for topical application and for the treatment of *genital herpes* and other inflammation of the genitals comprising as active substances tea tree oil, pau d'arco powder (an extract of *lapacho*), liquorice root extract and Echinacea extract with a local anaesthetic, preferably pramoxine hydrochloride. The composition may include soothing ingredients such as aloe vera extract and chamomile extract and it is preferably formulated in a cream base.

Another publication, US 2006/142271 A1, illustrates new synthetic derivatives of *lapacho* and their applications, in particular in pharmaceutical compositions for the treatment of cell proliferation disorders. These compounds can also be used in the treatment of psoriasis or cancer or precancerous conditions.

The publication US 2008/220106 A1 refers to toiletries, and in particular to otological solutions which basically contain extracts of *Lapacho* in polar or nonpolar solvents, and to the preparation thereof.

The publication WO 2017/060535 A1 relates to a topical herbal formulation which is particularly suitable for the treatment of wounds and a method for the production thereof. More specifically, it relates to a herbal formulation for the healing of topical wounds which comprises kotu cola (*Centella asiatica*)*,* greater plantain (*Plantago major*)*,* figwort (*Scrophularia nodosa*)*,* yarrow (*Achillea millefolium*) and Pau d'Arco (*Tabebuia impetiginosa*)*.* The herbal formulation has anti-inflammatory and antimicrobial properties. The formulation is particularly effective in the treatment of horse wounds, preventing tuberculosis or the formation of glabrous scars and favouring hair growth in the horse without the need for the introduction of an extracellular matrix. The formulation is also suitable for the treatment of general skin disorders in humans including but not limited to eczema and diaper rash.

### SUMMARY OF THE INVENTION

A method capable to extract and isolate long-chain fatty acids, from C16 to C24, in particular unsaturated ones, from the bark and inner parts of the bark, but also from leaves, flowers, buds and other parts of *Tabebuia impetiginosa (Lapacho* or *Pau d'arco)* has now been surprisingly found, for food, nutraceutical, dietary use and therapeutic applications. The invention refers to fractions isolated and purified from the bark and inner parts thereof of *Tabebuia impetiginosa* by a specific process of extraction and particularly rich in monounsaturated and polyunsaturated fatty acids defined omega and essential.

The present method turned out to be more efficient compared to what is known in the state of the art as it succeeds surprisingly to extract and purify a large amount of long-chain fatty acids, from C16 to C24, both saturated and unsaturated constituting the active fraction able to provide food intake and dietary supplements during specific treatments useful to balance and correct dyslipidaemias, metabolic disorders linked to lipid imbalances and altered cellular and tissue lipid compositions. Said method turned out to be surprisingly more efficient compared to the use of generic extracts based on aqueous or organic solvents or mixtures thereof. The obtained fraction, enriched in long-chain fatty acids, in particular both saturated and unsaturated, is particularly useful for food, nutraceutical, dietary use and for therapeutic applications.

### DETAILED DESCRIPTION OF PREFERRED EMBODIMENTS

In a first embodiment, the invention then provides an isolated fraction of *Tabebuia impetiginosa* enriched in long-chain fatty acids, from C16 to C24, both saturated and unsaturated in a percentage greater than 95%.

The isolated fraction of the invention made up of saturated fatty acids shows in particular a high percentage, greater than 25% by weight, of long-chain fatty acids C16 (palmitic acid), C18 (stearic acid) and C20 (arachidic acid).

The isolated fraction of the invention is characterized by a high percentage content, greater than 40% w/w, of monounsaturated long-chain fatty acids, in particular C16:1 (palmitoleic acid) and C18:1 (oleic acid).

The isolated fraction of the invention is characterized by a content of polyunsaturated fatty acids, in a percentage greater than 10%, in particular C18:2 (linoleic acid), C18:3 (alpha-linolenic acid), C20:3 (eicosatrienoic acid), C20:4 (arachidonic acid), C20:5 (eicosapentaenoic acid, EPA), C22:2 (docosadienoic acid), C22:5 (docosapentaenoic acid) and C22:6 (docosahexaenoic acid, DHA).

The isolated fraction of the invention consisting of polyunsaturated fatty acids is characterized by a content in omega-3 C18:3 (alpha-linolenic acid, ALA), C20:5 (eicosapentaenoic acid, EPA) and C22:6 (docosahexaenoic acid, DHA), in a percentage greater than 3%.

The isolated fraction of the invention consisting of polyunsaturated fatty acids is characterized by a content of C20:4 (arachidonic acid), in a percentage greater than 1.5% w/w.

The isolated fraction from *Tabebuia impetiginosa* comprising a content not lower than 95% w/w of long-chain fatty acids, from C16 to C24, both saturated and unsaturated (monounsaturated and polyunsaturated), omega-3 and essential, is particularly suitable for food, nutraceutical, dietary use in providing food intake and dietary supplements during specific treatments useful to balance and correct dyslipidaemias, metabolic disorders linked to lipid imbalances and altered cellular and tissue lipid compositions.

The method of the invention also relates to a process for the preparation of the fraction enriched in long-chain fatty acids, from C16 to C24, from *Tabebuia impetiginosa* comprising the steps of:
(a) drying in particular the bark and the inner parts of the bark of *Tabebuia impetiginosa;*
(b) breaking into fragments the bark and the inner parts of the bark, the leaves, the flowers, the buds and other parts of dried *Tabebuia impetiginosa* by a manual or mechanical crusher able to reduce the different parts in a fine powder consisting of particles having size not greater than 1000 micrometres;
(c) treating *Tabebuia impetiginosa* with water;
(d) subjecting the obtained solution to centrifugation in order to obtain a precipitate insoluble in aqueous phase;
(e) liquid phase removal and recovery of the insoluble phase;
(f) solubilizing the insoluble phase in aqueous phase obtained in point (e) in methanol-toluene in a well-defined ratio, in particular ranging between 75:25 and 65:35;
(g) mixing the solution;
(h) subjecting the obtained solution to centrifugation;
(i) precipitate removal and recovery of the liquid phase;
(j) further filtration of the liquid phase to remove any insoluble residues;
(k) subjecting the liquid phase obtained in the previous point to concentration and drying in order to obtain a white powder for use in finished preparations.

The invention also provides nutraceutical compositions and dietary supplements comprising the extract obtained by the above-mentioned method. The composition of the invention can be useful for food, nutraceutical, dietary use and for a range of therapeutic applications regarding dyslipidaemias, metabolic disorders linked to lipid imbalances and altered cellular and tissue lipid com positions.

As used in the present document, the term long-chain fatty acids, from C16 to C24, encompasses a heterogeneous family of compounds comprising aliphatic monocarboxylic acids mainly having a chain consisting of 16 to 24 carbon atoms, in an even number, branchless and acyclic, that is consisting of molecules which do not have loop chains. They have an aliphatic chain with very pronounced hydrophobic properties thanks to the length of the chain itself, unlike the short-chain fatty acids, and a carboxyl group able to establish ester bonds with various other biomolecules to build structurally and functionally more complex lipids which constitute important cellular constituents, such as the glycerophospholipids and the membrane sphingolipids, in addition to triglycerides.

As used in the present document, the term saturated fatty acids encompasses a group of fatty acids consisting of a carbon chain solely formed by single carbon-carbon bonds, therefore without the presence of double or triple carbon-carbon bonds. On the contrary, as used in the present document, the term unsaturated fatty acids encompasses a group of fatty acids consisting of a carbon chain wherein, besides single bonds, also carbon-carbon double bonds located in specific locations along the aliphatic hydrocarbon chain are present.

As used in the present document, the term monounsaturated fatty acids refers to a group of fatty acids consisting of a carbon chain wherein, beside single bonds, a unique double carbon-carbon bond located in specific position along the aliphatic hydrocarbon chain is present too.

As used in the present document, the term polyunsaturated fatty acids refers to a group of fatty acids consisting of a carbon chain wherein, beside single bonds, more than one double carbon-carbon bond, from two to six, located in specific locations along the aliphatic hydrocarbon chain are present too.

As used in the present document, the term "omega" fatty acids encompasses a group of polyunsaturated fatty acids that have important biological functions. The polyunsaturated fatty acids are therefore necessary for human beings and they can be divided mainly in omega-3, omega-6 and omega-9 fatty acids according to the position of the first double bond with respect to the terminal methyl group of the hydrocarbon chain. The main omega-3 fatty acids are alpha-linolenic acid (ALA), eicosapentaenoic acid (EPA), and docosahexaenoic acid (DHA). The double bonds are in cis position. The EPA and DHA acids can be synthesized by humans starting from ALA but only in small quantities. The precursor of omega-3 fatty acids is alpha-linolenic acid and it is the most abundant omega-3 fatty acid in western countries with an intake of 1.7 g against 135 mg of EPA and DHA.

As used in the present document, the term "essential" fatty acids encompasses a group of unsaturated fatty acids that are not biosynthesized in humans or are biosynthesized in small quantities not sufficient to ensure the necessary daily nutritional intake. In fact, the biosynthesis of long-chain omega-3 fatty acids such as EPA and DHA takes place mainly in the liver, in particular in peroxisomes and in the endoplasmic reticulum. The synthesis is divided into seven steps that involve the use of desaturases, enzymes that catalyse the introduction of double bonds, and elongases, which catalyse the elongation of the chain of the fatty acid. It should be emphasised that the production of omega-6 and omega-3 fatty acids follows two separate paths as an omega-3 fatty acid cannot be converted in an omega-6 and vice versa. However, enzymes participating in the metabolism of both fatty acids are the same, therefore they compete. In particular, the Δ6-desaturase enzyme is very important as it affects the speed of the biosynthesis of polyunsaturated fatty acids. Substances such as saturated fats, alcohol, glucocorticoids and adrenaline can inhibit the activity of the enzyme, just like noted with a reduction in the amount of desaturase in subjects affected by diabetes mellitus or subjected to ionizing radiation. The elderly and children also have a reduced omega-3 biosynthetic pathway so it is necessary to integrate with EPA and DHA.

As used in the present document, the terms "amount", "concentration" and "percentage" refer to the content of the different components fatty acids, expressed as a percentage by weight of the single biomolecules based on the total weight of the fraction of the present invention.

The method comprises the steps of:
(a) drying in particular the bark and the inner parts of the bark of *Tabebuia impetiginosa*;
(b) breaking into fragments the bark and the inner parts of the bark, the leaves, the flowers, the buds and other parts of dried *Tabebuia impetiginosa* by a manual or mechanical crusher able to turn the different parts in a fine powder consisting of particles having size not greater than 1000 micrometres;
(c) treating *Tabebuia impetiginosa* with water;
(d) subjecting the obtained solution to centrifugation in order to obtain a precipitate insoluble in aqueous phase;
(e) liquid phase removal and recovery of the insoluble phase;
(f) solubilizing the insoluble phase in aqueous phase obtained in the previous point in methanol-toluene in a ratio ranging between 75:25 and 65:35;
(g) mixing the solution;
(h) subjecting the obtained solution to centrifugation;
(i) precipitate removal and recovery of the liquid phase;
(j) further filtration of the liquid phase to remove any insoluble residues;
(k) subjecting the liquid phase obtained in the previous point to concentration and drying in order to obtain a white powder for use in finished preparations.

The fraction obtained in the step (j) or (k) is substantially rich in long-chain fatty acids, from C16 to C24, both saturated and unsaturated in a percentage greater than 95%.

The fraction obtained in the step (j) or (k) contains, among the long-chain saturated fatty acids, a high percentage of palmitic acid (C16), stearic acid (C18) and arachidic acid (C20).

The fraction obtained in the step (j) or (k) contains, among the long-chain monounsaturated fatty acids, a high percentage content of palmitoleic acid (C16:1) and oleic acid (C18:1).

The fraction obtained in the step (j) or (k) contains, among the long-chain polyunsaturated fatty acids, linoleic acid (C18:2), alpha-linolenic acid (ALA, C18:3), eicosatrienoic acid (C20:3), arachidonic acid (C20:4), eicosapentaenoic acid (EPA, C20:5), docosadienoic acid (C22:2), docosapentaenoic acid (C22:5) and docosahexaenoic acid (DHA, C22:6), in a percentage greater than 10%.

The fraction obtained in the step (j) or (k) of the present invention contains, among the long-chain polyunsaturated fatty acids, a percentage greater than 3% of omega-3 alpha-linolenic acid (ALA, C18:3), eicosapentaenoic acid (EPA, C20:5) and docosahexaenoic acid (DHA, C22:6).

The fraction obtained in the step (j) or (k) of the present invention contains, among the long-chain polyunsaturated fatty acids, a percentage greater than 1.5% of arachidonic acid (C20:4).

The composition of the fraction obtained in the step (j) or (k) of the present invention contains small percentages, lower than 5%, of fatty acids having a shorter chain, lower than or equal to C14, other glycosylated biocompounds known to be present in extracts of *Tabebuia impetiginosa* such as glycosides (consisting of four iridoid glycosides, two lignans glycosides, two isocoumarin glycosides, three phenylethanoid glycosides and eight phenol glycosides), and other phytomolecules such as furanonaphthoquinones, naphthoquinones, anthraquinones, quinones, benzoic acid, flavonoids, cyclopentene dialdehydes and other derivatives, coumarins, phenol glycosides, which are not soluble or are weakly soluble in solvents and in solvent ratios as used and described in the present invention.

As an alternative or together with the bark and the inner parts of the bark of *Tabebuia impetiginosa* other parts of the plant such as leaves, flowers, buds, and other portions of stem and roots can be used.

The method of the invention is hereinafter disclosed in detail in the present document.

### Step 1. Preparation of the solution of the raw material Tabebuia impetiginosa in a suitable solvent

i. The raw material *Tabebuia impetiginosa,* in particular the bark and the inner parts of the bark but other parts too such as leaves, flowers, buds and roots, are dried in an oven at not high temperatures, ranging between 20 and 40°C, preferably 25°C or at room temperature. Drying is carried out in conditions of low humidity, ranging between 40 and 70% of relative humidity, preferably between 50 and 60% for a period of not less than 24 hours, preferably for a time ranging between 30 and 40 hours.
ii. The dried raw material *Tabebuia impetiginosa* of point i. is crushed using preferably industrial crushers in order to obtain a fine powder consisting of particles having size ranging between 500 and 1000 micrometres, preferably ranging between 600 and 800 micrometres.
iii. The material of point ii. is sieved on sieves having porosity lower than 1000 micrometres in order to recover the sieved material of size lower than 1000 micrometres. The material remaining on the sieve is again crushed and sieved. The cycle is repeated until most raw material of *Tabebuia impetiginosa* shows a particle size lower than 1000 micrometres.
iv. The material of point iii. is dissolved in 100% water, preferably deionized water.
v. The raw material *Tabebuia impetiginosa* is dissolved in the solvent of point iv. at a concentration weight/volume from 20 mg/mL to 40 mg/mL, typically at a concentration of 30 mg/mL.
vi. The solution of *Tabebuia impetiginosa* of point v. is mixed under stirring at a temperature from 20°C to 30°C, preferably at room temperature ranging between 22°C and 25°C. The solution is stirred for a time from 10 to 20 hours, preferably from 13 to 17 hours.
vii. The solution of *Tabebuia impetiginosa* of point vi. is subsequently treated in an ultrasonic bath at a controlled temperature ranging between 20 and 30°C, preferably at 24°C, with a power from 200 to 500 Watt, preferably with a power ranging between 300 and 400 Watt for a time ranging between 10 and 20 hours, preferably for 15 hours.

### Step 2. Concentration of the fraction containing the long-chain fatty acids

i. The aqueous solution of point vii. is centrifuged to remove the insoluble material between 10000 and 20000 rpm, preferably 15000 rpm, at a temperature from 20°C to 25°C, preferably of 23°C, for 10 to 20 minutes, preferably 15 mins. The solution at point vii. can be filtered or further filtered after centrifugation on cellulose filters at a temperature from 25°C to 30°C, preferably of 28°C, a temperature slightly higher to facilitate gravity filtration. The temperature of the solution ranging between 25°C and 30°C is reached by heating the solution in a thermostatic bath before carrying out filtration on cellulose filters.
ii. The liquid phase of the previous point i. is discarded and the insoluble phase obtained by centrifugation or filtration or both is recovered. The insoluble phase of the previous point i. is enriched in long-chain fatty acids but still contains a high percentage, generally ranging between 20 and 50% w/w of other biomolecular components, among which short-chain fatty acids, depending on the type of material from *Tabebuia impetiginosa* used in point i. of Step 1.

### Step 3. Separation of long-chain fatty acids by liquid-liquid partition

i. The insoluble phase obtained by centrifugation or filtration or both of the previous point ii. is dried at low temperature, between 25 and 35°C, preferably at 30°C for a maximum of 24 hours, preferably for a time ranging between 12 and 15 hours.
ii. The dried insoluble phase of the previous point i. is solubilised in methanol-toluene in a ratio between 75:25 and 65:35, preferably 70:30 v/v at a concentration weight/volume between 10 mg/mL and 20 mg/mL, typically at a concentration of 15 mg/mL.
   The inventors have actually surprisingly found that the new method of the invention, which provides the described solubilization step in methanol-toluene in a ratio between 75:25 and 65:35, allows surprisingly to isolate a fraction of *Tabebuia impetiginosa* rich in long-chain fatty acids, from C16 to C24, saturated and unsaturated, in a percentage greater than 95% by weight.
iii. The solution obtained of the previous point ii. is mixed under slow stirring at low temperature ranging between 20°C and 25°C, preferably at room temperature of 22°C. The solution is stirred under the conditions described above for a maximum period of 20 hours, preferably from 10 to 16 hours, typically for 12 hours.
iv. The solution of point (iii) is centrifuged in order to remove the insoluble material between 5000 and 10000 rpm, preferably 8000 rpm, at a temperature between 2°C and 8°C, preferably of 5°C, for 15 to 30 minutes, preferably 20 mins.
v. The insoluble phase obtained by centrifugation of the previous point iv. is discarded and the soluble phase enriched in long-chain fatty acids and poor in other biomolecular components present in *Tabebuia impetiginosa* is recovered.
vi. The soluble phase in methanol-toluene of the previous point v. is further cleaned from insoluble or poorly soluble material by filtration on cellulose filters at low temperature ranging between 5°C and 15 °C, preferably of 10°C.
vii. The clear solution obtained from the filtration at the previous point vi., free from insoluble or poorly soluble material, is recovered.

### Step 4. Drying of the extract of Tabebuia impetiginosa rich in long-chain fatty acids

i. The solution of the previous point vii., rich in long-chain fatty acids and poor in other biomolecular components present in *Tabebuia impetiginosa,* is concentrated and dried by evaporation of the solvent at low temperatures, ranging between 20 and 30°C, preferably at 25°C, because higher temperatures induce the partial oxidation of long-chain unsaturated fatty acids. In a preferred embodiment, drying using rotary evaporators at low temperature, not higher than 30°C, or freeze drying with freeze dryers suitable for freeze-drying organic solutions, or a combination thereof, is used.
ii. The dry extract obtained at the previous point i. in the form of a white powder, forms the active part substantially rich in long-chain fatty acids, from C16 to C24, both saturated and unsaturated in a percentage greater than 95%.
iii. The object of the present invention can be used as such or solubilized in a mixture of organic solvents or also in the presence of small volumes of aqueous solvent to produce active liquid products.

The dry product of point iii. has been analysed by gas-chromatography (GC)-FID (flame ionisation detector) and GC-MS (single quadrupole mass spectrometer) for the content and the presence of long-chain saturated and unsaturated fatty acids.

GC equipped with two FID and MS detectors, by the usage of fused-silica capillaries 30 meters long and gaseous phases, is able to separate long-chain fatty acids based on the length of their hydrocarbon chain and therefore on their hydrophobicity and on the basis of the absence or presence of double bonds, and therefore on their molecular structure. In addition, the mass detector is able to identify the different molecules on the basis of their mass values in addition to the use of widely commercially available reference standards.

As fully described in the examples, the dry product obtained from the present invention by liquid-liquid partition, contains long-chain fatty acids, from C16 to C24, both saturated and unsaturated, in a percentage greater than 95%.

The isolated fraction of the invention consisting of saturated fatty acids also features a high percentage of long-chain fatty acids C16 (palmitic acid), C18 (stearic acid) and C20 (arachidic acid), in a percentage greater than 25% w/w.

The product of the invention is also characterized by a high percentage of long-chain monounsaturated fatty acids, in particular C16:1 (palmitoleic acid) and C18:1 (oleic acid), in amounts in a percentage greater than 40%.

The isolated fraction of the invention is still characterized by a content in polyunsaturated fatty acids, in a percentage greater than 10%, in particular C18:2 (linoleic acid), C18:3 (alpha-linolenic acid), C20:3 (eicosatrienoic acid), C20:4 (arachidonic acid), C20:5 (eicosapentaenoic acid, EPA), C22:2 (docosadienoic acid), C22:5 (docosapentaenoic acid) and C22:6 (docosahexaenoic acid, DHA).

The isolated fraction of the invention consisting of polyunsaturated fatty acids is also characterized by a content in omega-3 C18:3 (alpha-linolenic acid, ALA), C20:5 (eicosapentaenoic acid, EPA) and C22:6 (docosahexaenoic acid, DHA), in a percentage greater than 3%.

Finally, the isolated fraction of the present invention consisting of polyunsaturated fatty acids is characterized by a content in C20:4 (arachidonic acid), in a percentage greater than 1.5%.

The isolated fraction from *Tabebuia impetiginosa* comprising a content not less than 95% weight/weight of long-chain fatty acids, from C16 to C24, both saturated and unsaturated (monounsaturated and polyunsaturated), omega-3 and essential, is therefore particularly suitable for food, nutraceutical, dietary use in providing food intake and dietary supplements during specific treatments useful to balance and correct dyslipidaemias, metabolic disorders linked to lipid imbalances and altered cellular and tissue lipid compositions.

Still using GC-FID and GC-MS, the dry product of point iii. obtained at the end of the process described in the present invention contains small percentages, always lower than 5%, of fatty acids having a shorter chain, lower than or equal to C14. The fraction lower than 5% present in the dry product of point iii. also contains, beside the short-chain fatty acids, lower than or equal to C14, other small percentages of biomolecules, in particular glycosylated derivatives known to be present in the plant of *Tabebuia impetiginosa.* However, these biomolecules are not soluble or are weakly soluble in solvents and in solvent ratios as used and described in the present invention and therefore present in minimum percentages, collectively, together with the short-chain fatty acids, lower than 5% w/w.

The product obtained by the above-described method can be suitably formulated, in a mixture with an acceptable carrier in a form suitable for the administration by oral, topical, parenteral or transdermal route, for cosmetic, dermatologic, pharmaceutical or nutraceutical applications.

A topical formulation may be suitable for dermal, vaginal, rectal administration, for inhalation, intranasal, ocular, auricular or buccal administration. Thanks to the presence of long hydrocarbon chains and to their marked hydrophobicity, examples of suitable carriers include a mineral oil or a vegetable oil or a combination thereof. The carrier may also include at least a wax, such as beeswax, vegetable waxes, sugar cane waxes, mineral waxes and synthetic waxes.

Examples of suitable forms include capsules, tablets, liposomes, suppositories, suspensions, ointments, creams, lotions, solutions, emulsions, nanoemulsions, microemulsions, films, cements, powders, glues, aerosols, sprays.

The fraction of the present invention is rich in long-chain fatty acids in a percentage greater than 95% w/w in a balanced composition wherein saturated fatty acids are present that can be used not only to form the biological membranes but also for the production of energy, monounsaturated fatty acids needed for the proper functioning of the biological membranes making these capable of movements and membrane fluidity, polyunsaturated fatty acids, among which an important percentage of omega-3 fatty acids, indispensable for the formation of complex lipids, such as phosphosphingolipids and glycosphingolipids among which the gangliosides, which form the neuronal membranes and present in the nervous central system and, finally, an useful amount of arachidonic acid which represents a precursor of important functional biomolecules with local hormonal action, such as leukotrienes, thromboxanes and prostaglandins.

The therapeutically effective amount of the product of the invention may vary according to the recipient, the route and the frequency of administration. In general, the amount can suitably be in the range from about 1% to about 90% w/w compared with the total weight of the composition.

The pharmaceutical or nutraceutical compositions of the invention can be produced by conventional mixing processes, granulation, soft gel encapsulation, dissolution, extraction or freeze drying, and solutions.

The invention is described in more detail in the following examples, wherein:
Figure 1 is the chromatogram of the fatty acids present in the fraction obtained according to the process described in the Example 1;
Figure 2 is the chromatogram of the fatty acids present in the fraction obtained according to the process described in the Example 2.

### Example 1

The bark of *Tabebuia impetiginosa* and the inner portion thereof is dried in an oven at 30°C at a relative humidity of 50% for 40 hours. The dry material is crushed by mechanical crushing followed by sieving on sieves having a size lower than 1000 micrometres, in order to obtain a powder consisting of particles of size ranging between 600 and 1000 micrometres. The cycle is repeated until most of the bark of *Tabebuia impetiginosa* is recovered as a result of the sieving with a particle size lower than 1000 micrometres.

The material dried and reduced in a size lower than 1000 micrometres, is solubilised in 100% water at a concentration of 20 mg/mL. Specifically, 2000 mg of dry material of *Tabebuia impetiginosa* in 100 mL of water are mixed under continuous stirring at room temperature of 25°C for 20 hours. After 20 hours of continuous mixing, the solution is transferred to an ultrasonic bath and treated at 25°C with a power of 300 Watt for further 10 hours.

The obtained solution is centrifuged at 15000 rpm, at 20°C, for 15 minutes and subsequently gravity filtered on a cellulose filter at a temperature of 25°C.

Following the centrifugation step, the liquid phase of the previous solution is discarded while the insoluble material in aqueous phase is recovered. At this point of the process, the insoluble material in aqueous phase deriving from the bark of *Tabebuia impetiginosa* is recovered in a new vessel.

The insoluble material in aqueous phase recovered from the previous step by centrifugation is air dried at 24°C for 15 hours.

This previously obtained material, now dry, is solubilised in a solution of methanol-toluene in a 70:30 v/v ratio at a concentration of 10 mg/mL. Specifically, 400 mg in 40 mL of methanol-toluene 70:30 v/v are mixed under slow stirring at room temperature of 23°C for 10 hours.

After 10 hours of continuous mixing, the solution is centrifuged at 5000 rpm in a centrifuge cooled at 5°C, for 20 minutes, in order to separate the organic phase consisting of methanol-toluene from a pellet insoluble in such a solvent.

The insoluble phase recovered by centrifugation in the previous point is discarded.

The organic phase consisting of methanol-toluene and recovered by centrifugation is further cleaned from any microparticles still insoluble or poorly soluble by filtration on nitrocellulose filters at a temperature of 6°C, a temperature obtained by putting the solution in a refrigerator.

At this point, the solution consisting of methanol-toluene 70:30 v/v and rich in long-chain fatty acids in a percentage greater than 95% is recovered.

The solution obtained from the steps of the invention set out above is dried by a rotary evaporator at a temperature of 25°C until a white powder is obtained. Specifically, the duration of drying to obtain the finished product in its dry form under the drying conditions set out above turned out to be of 22 hours.

Specifically, 130 mg of the fraction of the invention, corresponding to a recovery of 6.5%, are obtained.

About 5 mg of the fraction of the invention are derivatized to obtain the derivatives of the fatty acids transesterified in the respective methyl esters, which show higher volatility and lower polarity compared to the corresponding free fatty acids. The transesterified fatty acids are injected in a GC from the company Shimadzu Italia equipped with a flame ionisation detector and the various fatty acids separated on a capillary of 30 meters.

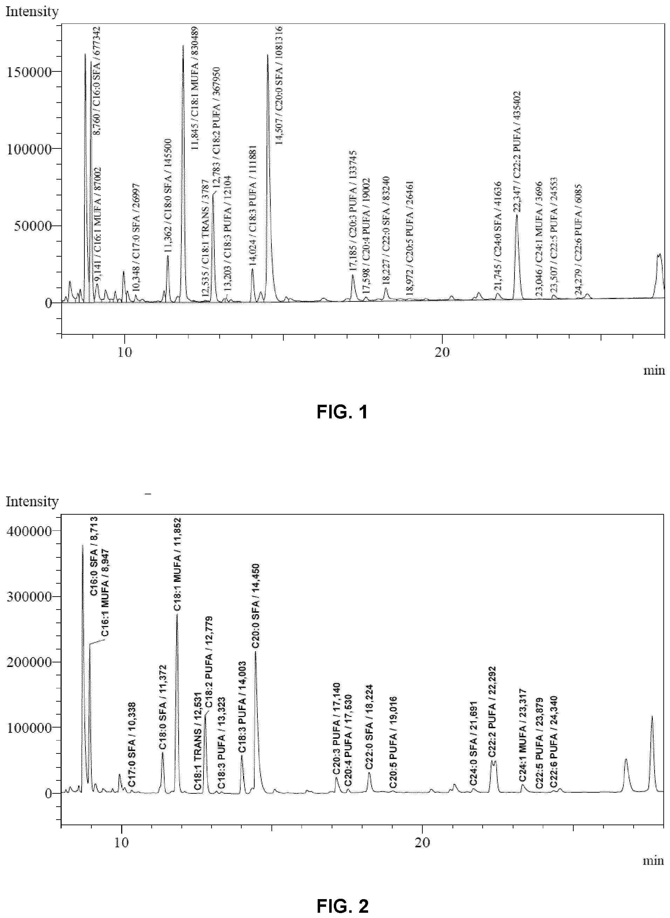

In Figure 1 a typical chromatogram of the fatty acids present in the fraction obtained according to the process described in the Example 1 is shown.

In Table 1 the fatty acids and their percentages present in the fraction of the invention obtained in the Example 1 and identified and quantified by the FID detector are reported.

| **Naming** | **Fatty acids** | **% w/w** |
|---|---|---|
| C16:0 | Palmitic | 14.3 |
| C16:1 | Palmitoleic | 32.9 |
| C17:0 | Margaric | 0.7 |
| C18:0 | Stearic | 7.6 |
| C18:1 | Oleic | 11.7 |
| C18:1 | Vaccenic | 1.9 |
| C18:2 | Linoleic | 2.8 |
| C18:3 | qamma-Linolenic | 0.2 |
| C18:3 | alpha-Linolenic | 1.0 |
| C20:0 | Arachidic | 5.8 |
| C20:3 | Eicosatrienoic | 0.5 |
| C20:4 | Arachidonic | 1.9 |
| C22:0 | Behenic | 1.6 |
| C20:5 | Eicosapentaenoic (EPA) | 2.6 |
| C24:0 | Liqnoceric | 3.7 |
| C22:2 | Docosadienoic | 1.5 |
| C24:1 | Nervonic | 1.3 |
| C22:5 | Docosapentaenoic | 2,6 |
| C22:6 | Docosahexaenoic (DHA) | 1.2 |
| Total | | **95.7** |
| | Palmitic + Stearic + Arachidic | **27.6** |
| | Palmitoleic + Oleic + Nervonic | **45.9** |
| | Linoleic + alpha-Linolenic + Eicosatrienoic + Arachidonic + EPA + Docosadienoic + Docosapentenoic + DHA | **14.0** |
| | alpha-Linolenic + EPA + DHA | **4.7** |
| | Arachidonic | **1.9** |

Table 1. Long-chain fatty acids and amounts thereof present in the dry fraction of *Tabebuia impetiginosa* present in the product obtained by the present invention in the Example 1 and identified and quantified by GC-FID and GC-MS.

As evident from Table 1, the fraction of the present invention produced in the form of a white powder contains long-chain fatty acids, from C16 to C24, in a concentration of 95.7% by weight.

The portion of saturated fatty acids palmitic acid + stearic acid + arachidic acid (C16 + C18 + C20) forms 27.6% w/w of the total of fatty acids of the fraction obtained from the present invention.

The portion of monounsaturated fatty acids with cis double bond palmitoleic acid + oleic acid + nervonic acid (C16:1 + C18:1 + C24:1) represents 45.9% w/w of the product of the present invention.

Instead, the percentage of long-chain polyunsaturated fatty acids linoleic acid + alpha-linolenic acid + eicosatrienoic acid + arachidonic acid + EPA + docosadienoic acid + docosapentenoic acid + DHA (C18:3 + C20:3 + C20:4 + C20:5 + C22:2 + C22:6 + C22:5) is present for 14.0% w/w.

The polyunsaturated fatty acids omega-3, alpha-linolenic + EPA + DHA (C18:3 + C20:5 + C22:6) of the fraction obtained from the process described in the present invention form 4.7% w/w.

The arachidonic acid (C20:4) is present in a w/w percentage of 1.9%.

In conclusion, the dry fraction in the form of a white powder obtained from the present method of liquid-liquid partition contains long-chain fatty acids, from C16 to C24, in a concentration greater than 95% by weight. The portion of saturated fatty acids is greater than 25% while the portion of monounsaturated fatty acids with double bond in cis represents a percentage greater than 40% by weight. The fraction consisting of polyunsaturated fatty acids forms a percentage greater than 10% of the total of fatty acids, and the omega-3s, biofunctional fatty acids of great nutritional and therapeutic importance, are present in a percentage by weight greater than 3% and it is made up of ALA, EPA and DHA. The arachidonic acid, important tetraunsaturated fatty acid C20:4, precursor of important cellular mediators and autocrine local hormones, of inflammatory processes, vasoconstrictors and bronchoregulators such as leukotrienes, thromboxanes and prostaglandins, is present in a percentage greater than 1.5% w/w.

The dry fraction of the present invention is rich in long-chain fatty acids in a balanced composition wherein the following are present: saturated fatty acids useful for the energy production, monounsaturated fatty acids needed for the proper functioning of biological membranes, polyunsaturated fatty acids, among which an important percentage of omega-3s, indispensable for the formation of complex lipids, such as the sphingolipids, which form the membranes of neurons and are present in the nervous central system, and, finally, a little but useful amount of arachidonic acid which represents a precursor of important functional biomolecules.

### Example 2

The inner part of the bark of *Tabebuia impetiginosa* scraped off from the same is dried in an oven at 40°C for the presence of a higher percentage of water at a relative humidity of 40% for 50 hours. The dry material is crushed by mechanical crushing followed by sieving on sieves having a size lower than 1000 micrometres. The cycle is repeated until most of the inner part of the bark of *Tabebuia impetiginosa* is recovered as a result of the sieving with a particle size lower than 1000 micrometres.

The material dried and reduced to a size lower than 1000 micrometres is solubilised in distilled water 100% at a concentration of 30 mg/mL. Specifically, 6000 mg of dry material of *Tabebuia impetiginosa* in 200 mL of water are mixed under continuous stirring at room temperature of 22°C for 15 hours. After 15 hours of continuous mixing, the solution is transferred to an ultrasonic bath and processed at 20°C with a power of 350 Watt for further 12 hours.

The obtained solution is centrifuged at 15000 rpm, at 20°C, for 20 minutes and then gravity filtered on a cellulose filter at a temperature of 22°C (room temperature).

Following the centrifugation step, the liquid phase of the preceding solution is discarded and the insoluble material in aqueous phase is recovered in a new vessel and air dried at 22°C (room temperature) for 24 hours.

The previously obtained material, now dried, is solubilised in a solution of methanol-toluene in a 75:25 v/v ratio at a concentration of 15 mg/mL. Specifically, 900 mg in 60 mL of methanol-toluene 75:25 v/v are mixed under slow stirring at room temperature of 22°C for 15 hours.

After 15 hours of continuous mixing, the solution is centrifuged at 10000 rpm in a centrifuge cooled at 5°C, for 30 minutes in order to separate the organic phase consisting of methanol-toluene from a pellet insoluble in such a solvent.

The insoluble phase recovered by centrifugation is discarded.

The organic phase consisting of methanol-toluene recovered by centrifugation is further cleaned from any microparticles still present by filtration on nitrocellulose filters at a temperature of 10°C, a temperature obtained by putting the solution in a refrigerator for a short while.

At this point, the solution consisting of methanol-toluene 75:25 v/v and rich in long-chain fatty acids is recovered.

The solution obtained from the previously described steps of the invention is processed in a rotary evaporator at a temperature of 20°C for 10 hours until a white paste is obtained, which is further dried by freeze drying with a freeze dryer equipped for organic solvents.

Specifically, in this Example 2, 420 mg of the fraction of the invention, corresponding to a recovery of 7.0%, are obtained.

About 5 mg of the fraction obtained in Example 2 are derivatized in order to obtain the derivatives of the fatty acids transesterified in the respective methyl esters and injected in the GC of the company Shimadzu Italia equipped with the FID detector to separate the various fatty acids.

In Figure 2 the chromatogram of the fatty acids present in the fraction obtained according to the process described in Example 2 is shown.

In Table 2 the fatty acids and their percentages present in the fraction of the invention obtained in Example 2 and identified by standard and mass spectrometer (MS) and quantified by the FID detector are reported.

| **Naming** | **Fatty acids** | **% w/w** |
|---|---|---|
| C16:0 | Palmitic | 16.6 |
| C16:1 | Palmitoleic | 31.3 |
| C17:0 | Marqaric | 1.6 |
| C18:0 | Stearic | 6.5 |
| C18:1 | Oleic | 10.1 |
| C18:1 | Vaccenic | 1.4 |
| C18:2 | Linoleic | 1.6 |
| C18:3 | qamma-Linolenic | 0.1 |
| C18:3 | alpha-Linolenic | 2.1 |
| C20:0 | Arachidic | 5.6 |
| C20:3 | Eicosatrienoic | 2.1 |
| C20:4 | Arachidonic | 1.9 |
| C22:0 | Behenic | 1.4 |
| C20:5 | Eicosapentaenoic (EPA) | 0.3 |
| C24:0 | Liqnoceric | 2.5 |
| C22:2 | Docosadienoic | 1.6 |
| C24:1 | Nervonic | 2.7 |
| C22:5 | Docosapentaenoic | 6.1 |
| C22:6 | Docosahexaenoic (DHA) | 1.4 |
| Total | | **97.0** |
| | Palmitic + Stearic + Arachidic | **28.7** |
| | Palmitoleic + Oleic + Nervonic | **44.1** |
| | Linoleic + alpha-Linolenic + Eicosatrienoic + Arachidonic + EPA + Docosadienoic + Docosapentenoic + DHA | **17.1** |
| | alpha-Linolenic + EPA + DHA | **3.8** |
| | Arachidonic | **1.9** |

Table 2. Long-chain fatty acids and their amounts present in the dry fraction of *Tabebuia impetiginosa* present in the product obtained in Example 2 and identified by standard and mass spectrometer (MS) and quantified by GC-FID and GC-MS.

As evident from Table 2, the fraction of the present invention produced in the form of a white powder contains long-chain fatty acids, from C16 to C24, in a concentration of 97.0% w/w.

The portion of the saturated fatty acids palmitic acid + stearic acid + arachidic acid (C16 + C18 + C20) is 28.7% w/w of the total of fatty acids of the fraction obtained from the present invention while the portion of monounsaturated fatty acids with cis double bond palmitoleic acid + oleic acid + nervonic acid (C16:1 + C18:1 + C24:1) represents 44.1% w/w.

The percentage of the long-chain polyunsaturated fatty acids linoleic acid + alpha-linolenic acid + eicosatrienoic acid + arachidonic acid + EPA + docosadienoic acid + docosapentenoic acid + DHA (C18:3 + C20:3 + C20:4 + C20:5 + C22:2 + C22:6 + C22:5) is present for 17.1% w/w and the polyunsaturated fatty acids omega-3 alpha-linolenic + EPA + DHA (C18:3 + C20:5 + C22:6) form 3.8% by weight. Finally, arachidonic acid (C20:4) is present in a percentage of 1.9%.

Also in this Example 2, the dry fraction obtained from the present method by liquid-liquid partition contains long-chain fatty acids, from C16 to C24, in a concentration greater than 95% by weight. The portion of saturated fatty acids is greater than 25% and the portion of monounsaturated fatty acids with double bond in cis represents a percentage greater than 40%. The polyunsaturated fatty acids are in a percentage greater than 10% of the total of fatty acids and omega-3s are present in a percentage greater than 3.0%. The arachidonic acid, also in this Example 2, is present in a percentage greater than 1.5%.

### Example 3

The bark and the inner portion thereof of *Tabebuia impetiginosa* is dried in an oven at 40°C at a relative humidity of 45% for 50 hours. The dry material is crushed as in the previous two examples by mechanical crushing and sieving to obtain particles having a size lower than 1000 micrometres.

The obtained material is solubilised in 100% distilled water at a concentration of 35 mg/mL. Specifically, 3500 mg of the dry material of *Tabebuia impetiginosa* in 100 mL of water are mixed under continuous stirring at room temperature for 15 hours and then transferred to an ultrasonic bath and processed at room temperature at 400 Watt for further 10 hours.

The obtained solution is centrifuged at 10000 rpm, at 20°C, for 30 minutes and then gravity filtered on a cellulose filter at room temperature. The produced liquid phase is discarded and the insoluble material is recovered and air dried at 24°C (room temperature) for 20 hours.

The material now dry is solubilised in methanol-toluene in a 65:35 v/v ratio at a concentration of 20 mg/mL. Specifically, 720 mg in 36 mL of methanol-toluene 65:35 v/v are mixed at room temperature for 16 hours.

The solution is then centrifuged at 10000 rpm at 5°C for 25 minutes in order to separate the organic phase consisting of methanol-toluene from a pellet insoluble in such a solvent.

The insoluble phase is discarded while the organic phase is filtered on nitrocellulose filters at a temperature of 6°C. The obtained solution is then dried in a rotary evaporator for 8 hours and subsequent freeze drying with a freeze dryer equipped for organic solvents.

Specifically, in this Example 3, 273 mg, corresponding to a recovery of 7.8%, are obtained.

About 5 mg of the fraction obtained in this Example 3 are also derivatized and injected in GC-FID in order to separate and quantify the various fatty acids.

In Table 3 the fatty acids and their percentages present in the fraction of the invention obtained in Example 3 are reported.

| **Naming** | **Fatty acids** | **% w/w** |
|---|---|---|
| C16:0 | Palmitic | 15.9 |
| C16:1 | Palmitoleic | 21.9 |
| C17:0 | Marqaric | 1.4 |
| C18:0 | Stearic | 5.4 |
| C18:1 | Oleic | 18.1 |
| C18:1 | Vaccenic | 1.4 |
| C18:2 | Linoleic | 1.9 |
| C18:3 | qamma-Linolenic | 0.7 |
| C18:3 | alpha-Linolenic | 1.7 |
| C20:0 | Arachidic | 6.1 |
| C20:3 | Eicosatrienoic | 0.4 |
| C20:4 | Arachidonic | 1.8 |
| C22:0 | Behenic | 1.7 |
| C20:5 | Eicosapentaenoic (EPA) | 1.8 |
| C24:0 | Liqnoceric | 3.7 |
| C22:2 | Docosadienoic | 1.1 |
| C24:1 | Nervonic | 4.0 |
| C22:5 | Docosapentaenoic | 4.8 |
| C22:6 | Docosahexaenoic (DHA) | 2.2 |
| Total | | **96.0** |
| | Palmitic + Stearic + Arachidic | **27.4** |
| | Palmitoleic + Oleic + Nervonic | **43.9** |
| | Linoleic + alpha-Linolenic + Eicosatrienoic + Arachidonic + EPA + Docosadienoic + Docosapentenoic + DHA | **15.7** |
| | alpha-Linolenic + EPA + DHA | **5.8** |
| | Arachidonic | **1.8** |

Table 3. Long-chain fatty acids and their amounts present in the dry fraction of *Tabebuia impetiginosa* present in the product obtained in Example 3.

As illustrated by Table 3, the fraction produced of the present invention in the form of a white powder contains long-chain fatty acids, from C16 to C24, in a concentration of 96.0% w/w.

The portion of the saturated fatty acids palmitic acid + stearic acid + arachidic acid (C16 + C18 + C20) is 27.4% while the portion of the monounsaturated fatty acids with cis double bond, palmitoleic acid + oleic acid + nervonic acid (C16:1 + C18:1 + C24:1), represents 43.9%.

The percentage of the long-chain polyunsaturated fatty acids linoleic acid + alpha-linolenic acid + eicosatrienoic acid + arachidonic acid + EPA + docosadienoic acid + docosapentenoic acid + DHA (C18:3 + C20:3 + C20:4 + C20:5 + C22:2 + C22:6 + C22:5) is present for 15.7% and the omega-3s alpha-linolenic acid + EPA + DHA (C18:3 + C20:5 + C22:6) form 5.8% by weight. The arachidonic acid (C20:4) is present in a percentage of 1.8%.

Also in this Example 3, the fraction obtained from the present invention by liquid-liquid partition contains long-chain fatty acids, from C16 to C24, in a concentration greater than 95% by weight. The portion of saturated fatty acids is still greater than 25% and the portion of monounsaturated fatty acids with double bond in cis represents a percentage greater than 40%. The polyunsaturated fatty acids are in a percentage greater than 10% and the omega-3s are present in a percentage greater than 3.0%. The arachidonic acid, also in this Example, is present in a percentage greater than 1.5%.

By way of comparison the following Table is provided in which the results of the method of the invention, in terms of percentage of long-chain saturated and unsaturated fatty acids from C16 to C24, in a fraction isolated from *Tabebuia impetiginosa,* have been compared with similar procedures of the prior art where, instead of the methanol-toluene solvent in a ratio ranging between 75:25 and 65:35, different solvents are used:

| **SOLVENTS** | **C16 - C24 ACIDS % w/w** |
|---|---|
| **Methanol/toluene (Ex. 1 invention)** | **95.7** |
| Petroleum ether | 14.9 |
| Ethyl acetate | 13.3 |
| Dichloromethane | 21.8 |
| Ethyl acetate / Dichloromethane | 13.4 |
| Ethanol | 12.2 |
| Methanol | 9.9 |
| Hexane | 14.7 |
| Chloroform | 6.7 |
| Butanol | 6.9 |

In this Table it is shown that the percentage of fatty acids, extracted from *Tabebuia impetiginosa* with the solvents of the prior art, is always lower than that of the invention, whose outcome is surprising and unexpected.

## Claims

1. Method for the preparation of a fraction isolated from *Tabebuia impetiginosa* rich in long-chain fatty acids, from C16 to C24, both saturated and unsaturated, in a percentage greater than 95% by weight, **characterized in that** it provides:
a) drying and fragmenting the parts of *Tabebuia impetiginosa* in order to obtain a fine powder consisting of particles having a size lower than 1000 micrometres;
b) treating the powder from *Tabebuia impetiginosa* with water;
c) subjecting the obtained solution to centrifugation in order to obtain a precipitate insoluble in the aqueous phase;
d) liquid phase removal and recovery of the insoluble phase;
e) solubilizing the insoluble phase obtained in the previous point in methanol-toluene in a ratio ranging between 75:25 and 65:35;
f) mixing the solution as obtained in point e);
g) subjecting the obtained solution to centrifugation;
h) precipitate removal and recovery of the liquid phase;
i) further filtration of the liquid phase for removing any insoluble residue;
j) subjecting the liquid phase obtained in the previous point to concentration and drying in order to obtain a white powder to be used in finished preparations.

2. Method according to claim 1, wherein the liquid phase in the concentration and drying step is removed by evaporation, freeze drying or a combination thereof.

3. Method according to claim 2, wherein the portion of long-chain fatty acids greater than 95% consists of saturated fatty acids in a percentage greater than 25% w/w.

4. Method according to claim 3, wherein the portion of saturated fatty acids in a percentage greater than 25% w/w consists of palmitic acid (C16), stearic acid (C18) and arachidic acid (C20).

5. Method according to claim 2, wherein the portion of long-chain fatty acids greater than 95% consists of monounsaturated fatty acids in a percentage greater than 40% w/w.

6. Method according to claim 5, wherein the portion of monounsaturated fatty acids in a percentage greater than 40% w/w consists of palmitoleic acid (C16:1) and oleic acid (C18:1).

7. Method according to claim 2, wherein the portion of long-chain fatty acids greater than 95% consists of polyunsaturated fatty acids in a percentage greater than 10% w/w.

8. Method according to claim 7, wherein the portion of polyunsaturated fatty acids in a percentage greater than 10% w/w consists of linoleic acid (C18:2), alpha-linolenic acid (C18:3), eicosatrienoic acid (C20:3), arachidonic acid (C20:4), eicosapentaenoic acid, EPA (C20:5), docosadienoic acid (C22:2), docosapentaenoic acid (C22:5) and docosahexaenoic acid, DHA (C22:6).

9. Method according to claim 7, wherein the portion of polyunsaturated fatty acids in a percentage greater than 10% w/w consists of a content in omega-3 alpha-linolenic acid, ALA (C18:3), eicosapentaenoic acid, EPA (C20:5) and docosahexaenoic acid, DHA (C22:6) in a percentage greater than 3.0%.

10. Method according to claim 7, wherein the portion of polyunsaturated fatty acids in a percentage greater than 10% w/w consists of a content in arachidonic acid (C20:4) in a percentage greater than 1.5% w/w.

11. Method according to claim 1, **characterized in that** said isolated fraction is obtained from the whole plant of *Tabebuia impetiginosa* or from selected parts thereof such as the bark and/or the inner portion thereof.

12. Food, nutraceutical, dietary, pharmaceutical, or cosmetic composition comprising the fractions obtained by the method according one or more of the preceding claims.

13. Composition according to claim 12, in the form of capsules, tablets, suppositories, suspensions, ointments, gels, creams, lotions, solutions, emulsions, films, powders, glues, aerosols, sprays.
